# EUROPEAN PATENT SPECIFICATION

(11) **EP 0 902 090 B1**
(45) Date of publication and mention of the grant of the patent: **12.11.2003**
(21) Application number: 98870191.8
(22) Date of filing: 11.09.1998
(51) Int. Cl.: C12N 15/88, C07C 257/14, A61K 9/127, A61K 48/00, A61K 47/48

(54) **Compound capable of introducing at least one molecule into a cell.**
Verbindung zum Einschleusen von mindestens einem Molekül in eine Zelle
Composé capable d'introduire au moins un molécule dans une cellule

(30) Priority: 12.09.1997 US 58636 P
(43) Date of publication of application: 17.03.1999
(73) Proprietor: BioTech Tools S.A., 1120 Bruxelles (BE)
(72) Inventor: Fuks, Robert, 1180 Brussels (BE); Ruysschaert, Jean-Marie, 1640 Brussels (BE); VandenBranden, Michel, 1170 Brussels (BE)
(74) Representative: Bird, William Edward

(56) References cited:
- WO-A-95/17378
- US-A- 3 879 460
- OUAHABI, A.E. ET AL.: "Double long-chain amidine liposome-mediated self replicating RNA transfection" FEBS LETTERS, vol. 380, 1996, pages 108-112, XP002089568
- RUYSSCHAERT, J-M. ET AL.: "A novel cationic amphiphile for transfection of mammalian cells" BBRC, vol. 203, no. 3, 1994, pages 1622-1628, XP002089569

## Description

### Field of the invention

The invention relates to a compound capable of introducing at least one molecule into a cell.

The invention also relates to a positively charged vesicle whose membrane comprises this compound, to a vector containing at least one molecule combined with the vesicle according to the invention; to the cell, animal and/or plant transformed by said compound or said vector as well as to the pharmaceutical or cosmetic composition, comprising said vector and/or the cell transformed by said vector.

The invention also relates to the process for the synthesis of said compound and to the process for the production of said vector.

Another aspect of the invention relates to the use of the compound and/or vector for the introduction *in vitro* and/or *in vivo* of at least one molecule into a cell.

### Technological background and prior state of the art which form the basis of the invention

Various processes are used in genetic engineering and/or in pharmacy to introduce molecules such as nucleic acids or active therapeutic agents into cells.

Transfection is a method which is widely used for introducing genetic material into cells, for studying the expression of genes, and for developing strategies for gene therapy.

Numerous experimental procedures using physical modifications (microinjection, electroporation and the like) or chemical modifications (dextran phosphate, calcium phosphate and the like) of membranes have been developed with varying success for introducing nucleic acids into cells using this method.

Another axis of research relates to the development of new amphiphilic cationic vectors which have demonstrated their effectiveness and their ease of use for causing the genetic material to penetrate into cells *in vitro* (P.L. Felgner et al., Proc. Natl. Acad. Sci., USA, 84, pp. 7413-7417 (1987)).

Some of these vectors such as the Lipofectin® N-(2,3-dioleyloxy)propyl-N,N,N-trimethylammonium chloride (DOTMA) from GIBCO BRL or the Transfection-reagent® N-(2,3-dioleoyloxy)propyl-N,N,N-trimethylammonium methyl sulfate (DOTAP) from Boehringer Mannheim GmbH, have been commercialized.

Patent Application WO91/15501 (YALE UNIVERSITY) also describes a positively charged reagent consisting of a neutral phospholipid such as dioleoylphosphatidylethanolamine and a cationic lipid such as stearylamine, a tertiary amine or a benzothorium salt for the transfection of nucleic acids.

Nevertheless, these products require large quantities of reagents in order to obtain an effective transfection of cells and therefore increase the cytotoxicity of the products and their cost (thus, the price of the "Lipofectin®" product (DOTMA) is US$ 145/ml, equivalent to US$ 10 per transfection).

In addition, Lipofectin® (DOTMA) has the additional disadvantage of not being capable of being added to a serum.

Like WO 95/17378, the document "Vesicle Formation by double long-chain Amidines" (Fabienne Defrise-Quertain et al.) (J. Chem. Soc., Chem. Commun., 1986, p. 1060 to 1062) describes the formation of vesicles consisting of 3-tetradecylamino-N-tert-butyl-N'-tetradecylopropionamidine.

However, this document does not contain sufficient description for the preparation of these compounds. Indeed, this document does not indicate the reaction temperatures to be used or the fact that it is necessary to use a basic aqueous solution in order to form the compound of formula 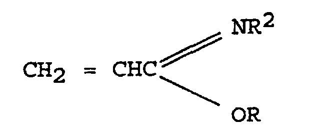 in which OR is the residue of an alcohol of formula ROH and R² is selected from the group comprising a hydrogen atom, saturated alkyl chains, unsaturated alkyl chains and substituted alkyl chains; an intermediate compound which is necessary for the production of the compounds according to the invention.

### Aims of the invention

The aim of the present invention is to produce a new compound and/or a new vector capable of introducing at least one molecule into a cell, without exhibiting the disadvantages of the prior state of the art.

The invention is also intended for producing a pharmaceutical or cosmetic composition comprising said vector and/or said cell transformed by said vector.

An additional aim of the present invention is intended for producing a vector which can be used in a serum.

### Characteristic elements of the invention

The present invention relates to a compound having an amidine polar head and the following formula : 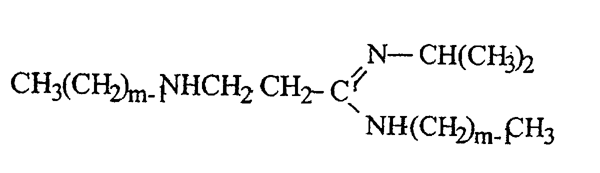 wherein m = 12, 14, 16 or 18.

The invention also relates to a positively charged vesicle having a membrane comprising a compound according to the invention as well as a vector consisting of said vesicle associated with at least one molecule to be introduced into a cell, said molecule being advantageously selected from the group consisting of nucleic acids (nucleic acids oligomers or nucleic polymers), antigens, polypeptides or optionally glycosylated proteins and/or active therapeutic or cosmetic agents.

Said molecule is introduced in a cell by a process wherein said cell is brought into contact with said molecule and a compound and/or a vesicle according to the invention. Likewise, the introduction process can be achieved by bringing said cell into contact with a vector according to the invention.

A "nucleic acid according to the invention" means any kind of nucleic acids polymer of oligomer such as a plasmid, messenger RNAs, antisense RNAs, cDNAs, synthetic oligonucleotides and the like which are capable of genetically transforming a cell and which may express a specific antigen, polypeptide or protein as well as an active therapeutic or cosmetic agent into said cell.

According to a first embodiment of the process of the invention, a cell is treated *in vitro* in order to produce plants or animals which are transgenic or for gene therapy, in particular for the treatment of cellular disorders such as cancer or infections such as viral or bacterial infections or the like.

According to the second preferred embodiment of the process of the invention, a cell is treated *in vivo* in order to produce recombinant microorganisms, plants and/or non-human animals which are transgenic or in for *ex vivo* or *in vivo* gene therapy.

Another aspect of the invention concerns a new composition comprising a molecule to compact DNA, preferably a protamine, a polylysine or a histone derived peptide, and the compound, the vesicle or the vector according to the invention.

The composition according to the invention may also comprise another compound which increases cell endocytosis, preferably a microtubule inhibitor such as colchicine.

Another aspect of the present invention is related to a transfection kit comprising said composition, compound(s), vesicle or vector according to the invention, for the transfection of cells, plants or animals.

The present invention also relates to the cell, the non-human animal or the plant transformed by the vector according to the invention as well as a cosmetic or pharmaceutical composition (such as a vaccine) comprising the vector and/or the transformed cell according to the invention.

Another aspect of the present disclosure relates to the process for the synthesis of the compound in which compounds of formula CH₂=CHC=N and R²-Cl are reacted with anhydrous ferric chloride so as to form the tetrachloroferrate of a compound of formula CH₂=CHC=N⁺-R², this tetrachloroferrate is treated with an amine of formula R³-NH-R⁴ so as to form a compound of formula :

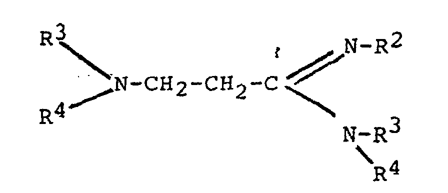

The invention also relates to a process for the production of the vector according to the invention in which at least one molecule selected from the group consisting of nucleic acids molecules, antigen molecules, polypeptides, glycosylated polypeptides, proteins, glycosylated proteins, therapeutic agents and cosmetic agents are reacted with a vesicle according to the invention.

A final aspect of the present invention relates to the use of the vector and/or the compound according to the invention for the introduction of one or more molecules into a cell *in vitro* as well as the use of the vector according to the invention for the introduction of one or more molecules into a cell *in vivo.*

### Examples

### Example 1 :

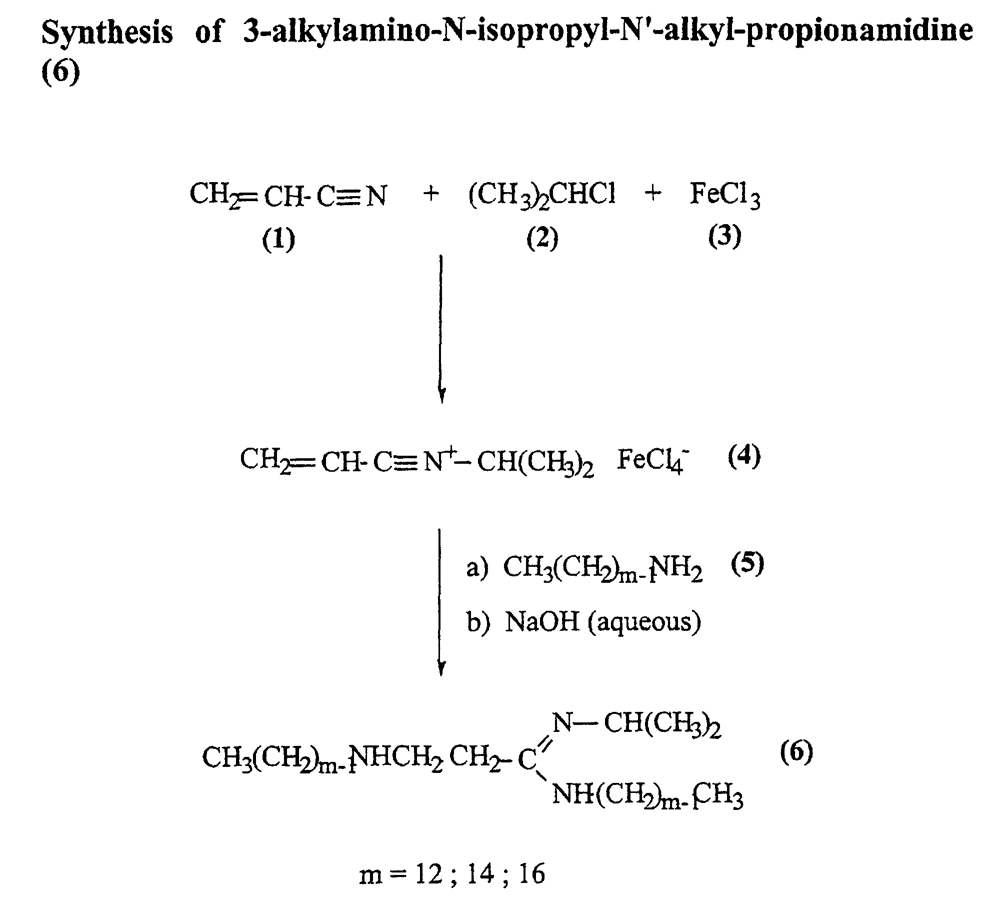

Acrylonitrile is converted to a nitrilium salt 4, which treated "in situ" with the alkylamine 5 gives in all cases the double-long-chain amidine 6 with yields depending on the chain length (see Table 1).

The following experimental procedure was used: 6,6 ml (0,1 mol) of acrylonitrile was added to 16,2 g (0,1 mol) of ferric chloride in 100 ml of isopropyl chloride cooled with an ice bath. The mixture was stirred under nitrogen for 1,5 hour. Excess isopropyl chloride was evaporated under vacuum. The residue, as represented by structure (4) was taken up in 100 ml of dichloromethane and the mixture cooled at -10 °C. Amine (5) (0,1 mol) in 150 ml of dichloromethane was added with stirring. After this addition, the mixture was kept overnight at room temperature with stirring. The mixture was then poored on 500 ml of 1,5 M aqueous sodium hydroxide cooled at 0 °C. The mixture is then separated after settling has taken place at room temperature. The aqueous phase is extracted twice with 100 ml of dichloromethane. The combined organic phases are dried over magnesium sulfate and then filtered. Evaporation of the solution gave the crude bouble long chain amino-amidine (5). It is taken up in hexane and filtered on an g-Tonerde alumina column. The filtrate is evaporated under vacuum and the residue crystallised from hexane in the refrigerator. It is recrystallised in the same solvent (see Table 1).

**Table 1 :**

| **Synthesis of 3-alkylamino-N-isopropyl-N'-alkylpropionamidine (6)** | | |
|---|---|---|
| **Alkylamine (5)** | **Hexane and Al**_{**2**}**O**_{**3**} | **Yield in amidine (6) after 4 crystallisations** |
| m=12 - 30.4g-0.164 mol | 50 ml - 200 g | 2.1 g - 9 % |
| m=14 - 35.0g-0.164 mol | 50 ml - 200 g | 3.1 g - 14 % |
| m=16 - 39.6g-0.164 mol | 50 ml - 200 g | 1.8 g - 6 % |

The physical, spectroscopic and analytical characteristics are given in Table 2.

**Table 2 :**

| **Physical, analytical and spectroscopic data of amidines (6)** | | | | |
|---|---|---|---|---|
| **amidine (6)** | **m.p. (°C)**^{**a**} | **molecular formula M** | **mass spectrum M**^{**+**}**·** | ^{**1**}**H NMR(CDCl**_{**3**} **; 250 MHz)** |
| m = 12 | 27 -28 | C₃₀H₆₃N₃ 465.85 | 465 | 3.73 (1H,s) ; 3.13 (2H,s) ; 2.79 (2H,t) |
| | | | | 2.58 (2H,t) ; 2.35 (2H,t) ; 1.47 (4H,m) |
| | | | | 1.26 (36H,s) ; 1.10 (6H,d) ; 0.88 (6H,t) |
| m = 14 | 29 - 30 | C₃₄H₇₁N₃ 521.96 | 521 | 3.73 (1H,s) ; 3.14(2H,s) ; 2.79 (2H,t) |
| | | | | 2.58 (2H,t) ; 2.34 (2H,t) ; 1.47 (4H, m) |
| | | | | 1.26 (44H,s) ; 1.10 (6H,d) ; 0.88 (6H,t) |
| m =16 | 38.5 - 39.5 | C₃₈H₇₉N₃ 578.06 | 577 | 3.74 (1H,s) ; 3.14 (2H,s) ; 2.78 (2H,t) |
| | | | | 2.58 (2H,t) ; 2.34 (2H,t) ; 1.46 (4H,m) |
| | | | | 1.26 (52H,s) ; 1.10 (6H,d) ; 0.88 (6H,t) |

### Example 2 : Formation of vesicles

The procedures for the formation of the vesicles are given for the two following compounds :
- 3-tetradecylamino-N-isopropyl-N'-tetradecylpropionamidine (6, m=14, diC14-isop-amidine (1 mg/ml), and
- 3-hexadecylamino-N-isopropyl-N'-hexadecylpropionamidine, (6, m =16, diC16-isop-amidine (1 mg/ml),
which are hereafter referred to as Vectamidine®.

### Procedure 1

A solution of Vectamidine is prepared in ethanol (50mg/ml). Dissolving is enabled by heating briefly at 55 °C. Twenty microliters of this solution are rapidly injected, by means of a micropippet, into 1ml of HBS buffer (10 mM Hepes, 150 mM NaCl, adjusted at pH 7.3 by addition of concentrated NaOH), heated to 55 °C. During the operation, the solution is shaken on a vortex mixer. The resulting vesicle suspension (named "the suspension") is incubated for an additional 1 minute at 55 °C and shaken again for 20 seconds on the vortex mixer. The suspension is allowed to cool at room temperature (20 °C) before use. In a large number of experiments, the suspension was stored at 4 °C for several weeks but heating at 55 °C for 5 minutes and vortexing was necessary to restore the full transfecting activity.

### Procedure 2

A solution of Vectamidine is prepared in ethanol (50mg/ml). Dissolving is enabled by heating briefly at 55 °C. If not used immediately, this solution is stored at -20 °C for better stability. Twenty microliters of this solution is placed in a lml propylene tube and ethanol is completely evaporated under a stream of dried nitrogen. One ml of HBS buffer is added and the tube is heated at 55 °C for 5 minutes. The tube is shaken vigorously on a vortex mixer for 20 seconds. The suspension is allowed to cool at room temperature (20 °C) before use.

### Example 3 : Transfection procedure

### Cell Lines

Transfection Procedures are given for the following cell lines : COS, CHO, BHK21, Hela, CV-1, NIH/3T3, HEK293.

Cells are mycoplasma-free, grow at their normal rate and show no morphological signs of degenerescence before use.

### Transfection medium

The transfection medium is defined as Dulbecco Modified Eagle Medium (DMEM) (Life Technologies 41965-039), without serum, without antibiotics with 20mM Hepes final conc. (from Hepes buffer, Life Technologies 15630-056).

### DNA solution

DNA is a pCMV-lacZ plasmid DNA reporter system. Efficient transfection was also demonstrated using other reporter system including pCMV-cat, pEGFP-N1(Clontech).

Plasmid DNA is obtained by the techniques commonly used in biotechnology laboratory: briefly, E. Coli (DH5alpha or MM298) are transformed with the plasmid and grown in L. B. medium and plasmid purification is performed using the Qiagen kit (Qiagen) according the instructions of the manufacturer.

Purity and integrity of the plasmid is checked by absorbance measurement at 260 and 280nm and agarose gel electrophoresis before use.

The final DNA solution is prepared by diluting plasmid DNA (from a lmg/ml stock solution in 10mM Tris, 1mM EDTA) in transfection medium to a concentration of 20µg/ml.

### Cell culture

Cells are routinely cultured in 75 cm² culture flasks (Orange Scientific, 2010200) in a cell incubator with 5% CO₂ and are passed every 2 days. The day before transfection, cells are briefly trypsinized (Trypsin-EDTA Life Technology 45300-027) and transfered to 96 wells plates (Orange Scientific, ref 2030100) at 1-2x10⁴ cells/well. Cell Status before transfection: 80% confluent after 24 hours culture.

### Transfection medium

DMEM (Life Technologies 41965-039), without serum, without antibiotics with 20mM Hepes final conc. (Life Technologies 15630-056).

### Preparation of the Vectamidine solutions

Vectamidine /DNA ratio have been tested according to the following table.

| **Vectamidine /DNA ratio** | **Volume of Vectamidine** | **Volume of transfection medium** |
|---|---|---|
| 1/1 | 5 microliters | 245 microliters |
| 2/1 | 10 microliters | 240 microliters |
| 3/1 | 15 microliters | 235 microliters |
| 4/1 | 20 microliters | 230 microliters |
| 6/1 | 30 microliters | 220 microliters |
| 8/1 | 40 microliters | 210 microliters |

The range shown in the table can be extended to other ratios if necessary.

### Vectamidine /DNA complexes formation

For each Vectamidine/DNA ratio mentioned in the table here-above, combine 250 µl of the corresponding Vectamidine solution with 250µl of DNA solution at 20 micrograms/ml in transfection medium into one sterile polystyrene tube and mix it gently but thoroughly.

Incubate the resulting Vectamidine /DNA complexes solution at room temperature for 15 min.

A few minutes before the Vectamidine /DNA complex formation is terminated, the medium is removed from the culture to be transfected and if cell adhesion is strong enough, the cells are washed with pre-warmed serum-free medium.

When the Vectamidine /DNA complexes are formed, dilute it 5 fold in transfection medium.

Gently aspirate the medium from culture vials and apply 100 microliters of the transfection solution onto the cells which have been grown in 96 wells plates.

Incubate culture plates at 37 °C in a CO₂ incubator for 30 min to 5 hours (2 to 3 hours were found optimal in most cases).

Completely remove the medium containing the transfection solution and replace with the complete medium recommended for the cell line under study. If cell adhesion is strong enough at this step, one can wash one time with complete medium. For suspended cells, proceed the washing step by centrifugation. Omitting this step and maintaining Vectamidine in culture medium after transfection may result in cell toxicity.

Culture cells in optimal conditions for growth and gene expression. For cells stopping growth while reaching confluence, cells are detached by trypsinization and transfer to larger dishes to allow optimal growth.

### Transfection efficiency measurement procedure (example given for lacZ reporter gene)

After 48 hours culture in culture medium for gene expression, cell supernatants are discarded and cells are lysed in lysis buffer (see below) for 15 minutes at room temperature. the β-galactosidase activity resulting from lacZ gene expression is measured using the ONPG colorimetric assay (see below).
***Lysis buffer:*** 0.5%Triton X100 (Merck 11869) and 0.1% Na deoxycholate (Sigma D-6750)
***ONPG assay in 96 wells microtiter plates:*** Serial 2 fold dilutions of cell lysates are made (minimum 4 dil). To 50µl of cell lysate per well, 50µl of ONPG Mix are added and color development is monitored at 420nm in a microtiter plate reader till color development. Avoid saturation. Keep protected from light between measurements. Reaction is terminated by the addition of 100µl of 1M Na2CO3.
***ONPG Mix (prepared before use):*** 0.2M Na phosphate pH7.0 (analytical grade), 2mM MgCl2 (analytical grade), 0.1M 2-mercaptoethanol (Sigma M-7154), 1.33mg/ml ONPG (2-nitrophenyl-β-D-galactopyranoside, Boerhinger Mannheim 810088)
***Standard curve:*** serial 2 fold dilutions of β-galactosidase (Boehringer Mannheim 105031, 1500U/ml) first dilution: 125mU/ml in lysis buffer, madebefore use.

### Transfection of K562 cells (suspension cells)

The general protocol of transfection given above is used except that all the cell washing and medium replacement steps are performed by transferring the cells in conical polystyrene tubes for centrifugation and resuspension in the desired medium. The K562 cells are cultured at 1 million cells in a 4 cm diameter dish before transfection. Two ml Vectamidine/DNA complex containing 5 micrograms plasmid and from 5 to 40 micrograms of Vectamidine are required for transfection of each 4 cm diameter dish. One dish is required for every transfection compound/DNA ratio tested.

### Transfection of HEK293 cells using 3-tetradecylamino-N-isopropyl -N'-tetradecylopropionamidine at different agent/DNA weight ratios indicated on the X-axis

The standard transfection protocol described hereabove was followed.

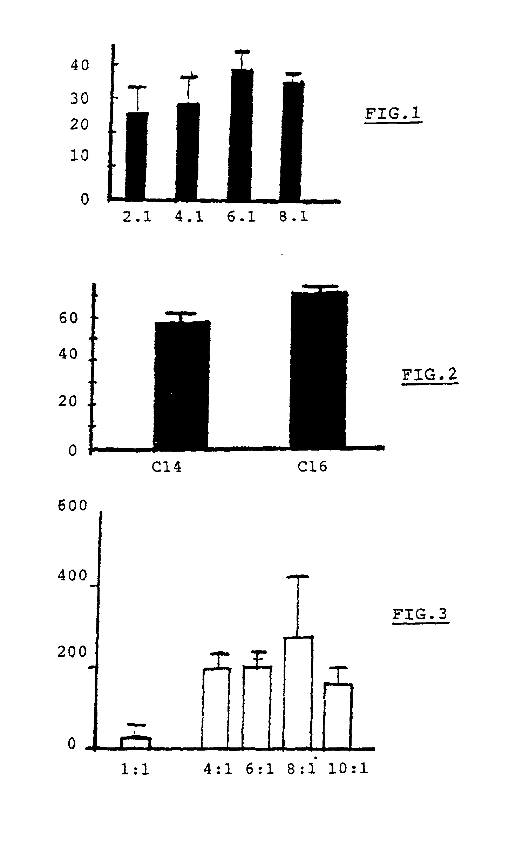

Transfection activity is indicated on the Y-scale (Beta-galactosidase units) of the Fig. 1.

### Transfection of HEK-293 cells using 3-tetradecylamino-N-isopropyl-N'-tetradecylpropionamidine (denoted C14 on the figure) and 3-hexadecylamino-N-isopropyl-N'-hexadecylaminopropionamidine (denoted C16 on the figure) at 6:1 agent/DNA weight ratios

The standard transfection protocol described hereabove was followed.

Transfection activity is indicated on the Y-scale (Beta-galactosidase units) of the Fig. 2.

### Transfection of COS cells using 3-tetradecylamino-N-isopropyl-N'-tetradecylopropionamidine at different agent/DNA weight ratios indicated on the X-axis

The standard transfection protocol described hreabove was followed.

Transfection activity is indicated on the Y-scale (Beta-galactosidase units) of the Fig. 3.

### Example 4 : Effect of the microtubule inhibitor on transfection efficiency, evaluated on BHK21 cells

The BHK cells were distributed at 0.2 10⁶ cells/well in 24 wells culture plate the day before transfection to reach an optimal confluency of 50-80% just before transfection.

Before transfection, cells are pre-incubated in 0; 1; 4 or 8 µM colchicine in complete Glasgow culture medium supplemented with 25 mM Hepes, 2 mM Glutamine, 5% foetal bovine serum. Colchicine is added from a DMSO stock solution taking care not to raise the final DMSO concentration to more than 1% vol. After 1 hour preincubation, cells are washed with serum-free medium and overlayed with 500 µl/well of serum-free medium containing colchicine at 0; 1; 4 or 8 µM and with a max 1% vol DMSO. The pCMV-lac Z (Clontech) was introduced into cells according to the transfection procedure.

Transfections were performed according to the above-described procedure and the enzymatic activity were measured according to the above-mentioned procedure.

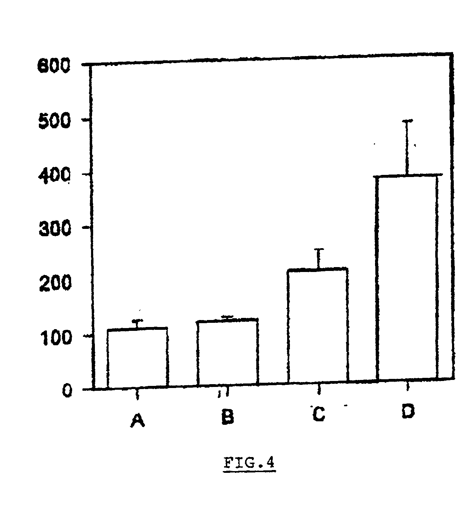

The Fig. 4 presents the transfection rate of the Lac I gene (expressed in mU/mg).

## Claims

1. A compound named 3-dodecylamino-N-isopropyl-N'-dodecyl-propionamidine.

2. A compound according to claim 1, having a melting point of 27-28°C.

3. A compound according to claim 1 or claim 2, having a ¹H NMR spectrum with characteristic peaks at 3.73, 3.13, 2.79, 2.58, 2.35, 1,47, 1.26, 1.10 and 0.88.

4. A compound named 3-tetradecylamino-N-isopropyl-N'-tetradecyl-propionamidine.

5. A compound according to claim 4, having a melting point of 29-30°C.

6. A compound according to claim 4 or claim 5, having a ¹H NMR spectrum with characteristic peaks at 3.73, 3.14, 2.79, 2.58, 2.34, 1.47, 1.26, 1.10 and 0.88.

7. A compound named 3-hexadecylamino-N-isopropyl-N'-hexadecyl-propionamidine.

8. A compound according to claim 7, having a melting point of 38.5-39.5°C.

9. A compound according to claim 7 or claim 8, having a ¹H NMR spectrum with characteristic peaks at 3.74, 3.14, 2.78, 2.58, 2.34, 1.46, 1.26, 1.10 and 0.88.

10. A compound named 3-octadecylamino-N-isopropyl-N'-octadecyl-propionamidine.

11. A positively charged vesicle having a membrane comprising a compound according to any of claims 1 to 10.

12. A vector consisting of a positively charged vesicle according to claim 11, said vesicle being associated with at least one molecule selected from the group consisting of nucleic acids, plasmids, messenger RNAs, antisense RNAs, cDNAs, synthetic oligonucleotides, polypeptides, proteins, glycosylated proteins, therapeutic agents and cosmetic agents.

13. Use of a vector according to claim 12 in a serum.

14. A process for introducing at least one molecule selected from the group consisting of nucleic acids, plasmids, messenger RNAs, antisense RNAs, cDNAs, synthetic oligonucleotides, antigens, polypeptides, proteins, glycosylated proteins, therapeutic agents and cosmetic agents into a cell, wherein said cell is brought into contact with said molecule and with a compound according to any of claims 1 to 10 or a positively charged vesicle according to claim 11 or a vector according to claim 12,
wherein the cell is treated *in vivo* to produce recombinant microorganisms, or plants.

15. The process of claim 14, wherein the cell is treated *in vitro.*

16. A cell transformed by a process according to any of claims 14 to 15.

17. A composition comprising a molecule to compact DNA and a compound according to any of claims 1 to 10 or a positively charged vesicle according to claim 11 or a vector according to claim 12.

18. A composition according to claim 17, wherein the said molecule is selected from the group consisting of protamines, polylysines and histone derived peptides.

19. A transfection kit comprising a composition according to claim 17 or claim 18.

20. A transgenic plant or non-human animal produced from a cell according to claim 16.

21. A pharmaceutical composition comprising a vector according to claim 12 or a cell according to claim 16.

22. A pharmaceutical composition according to claim 21, being a vaccine.

23. A pharmaceutical composition according to claim 21 for the treatment of cellular disorders or infections.

24. A cosmetic composition comprising a vector according to claim 12 or a cell according to claim 16.

## Patentansprüche

1. Verbindung mit der Bezeichnung 3-Dodecylamino-N-isopropyl-N'-dodecylpropionamidin.

2. Verbindung nach Anspruch 1 mit einem Schmelzpunkt von 27 - 28°C.

3. Verbindung nach Anspruch 1 oder Anspruch 2 mit einem ¹H NMR-Spektrum mit charakteristischen Peaks bei 3,73, 3,13, 2,79, 2,58, 2,35, 1,47, 1,26, 1,10 und 0,88.

4. Verbindung mit der Bezeichnung 3-Tetradecylamino-N-isopropyl-N'-tetradecylpropionamidin.

5. Verbindung nach Anspruch 4 mit einem Schmelzpunkt von 29 - 30°C.

6. Verbindung nach Anspruch 4 oder Anspruch 5 mit einem ¹H NMR-Spektrum mit charakteristischen Peaks bei 3,73, 3,14, 2,79, 2,58, 2,34, 1,47, 1,26, 1,10 und 0,88.

7. Verbindung mit der Bezeichnung 3-Hexadecylamino-N-isopropyl-N'-hexadecylpropionamidin.

8. Verbindung nach Anspruch 7 mit einem Schmelzpunkt von 38,5 - 39,5°C.

9. Verbindung nach Anspruch 7 oder Anspruch 8 mit einem ¹H NMR-Spektrum mit charakteristischen Peaks bei 3,74, 3,14, 2,78, 2,58, 2,34, 1,46, 1,26, 1,10 und 0,88.

10. Verbindung mit der Bezeichnung 3-Octadecylamino-N-isopropyl-N'-octadecylpropionamidin.

11. Positiv geladenes Vesikel mit einer Membran, enthaltend eine Verbindung nach einem der Ansprüche 1 - 10.

12. Vektor, bestehend aus einem positiv geladenen Vesikel nach Anspruch 11, wobei das Vesikel mit wenigstens einem Molekül assoziiert ist, welches ausgewählt wird aus der Gruppe, bestehend aus Nukleinsäuren, Plasmiden, Messenger RNAs, Antisense RNAs, cDNAs, synthetischen Oligonukleotiden, Polypeptiden, Proteinen, glykosilierten Proteinen, therapeutischen Mitteln und kosmetischen Mitteln.

13. Verwendung eines Vektors nach Anspruch 12 in einem Serum.

14. Verfahren zum Einführen wenigstens eines Moleküls, ausgewählt aus der Gruppe, bestehend aus Nukleinsäuren, Plasmiden, Messenger RNAs, Antisense RNAs, cDNAs, synthetischen Oligonukleotiden, Antigenen, Polypeptiden, Proteinen, glykosilierten Proteinen, therapeutischen Mitteln und kosmetischen Mitteln in eine Zelle, wobei die Zelle mit dem Molekül und mit einer Verbindung nach einem der Ansprüche 1 - 10 oder einem positiv geladenen Vesikel nach Anspruch 11 oder einem Vektor nach Anspruch 12 in Kontakt gebracht wird, wobei die Zelle in vivo behandelt wird, um rekombinante Mikroorganismen oder Pflanzen herzustellen.

15. Verfahren nach Anspruch 14, wobei die Zelle in vitro behandelt wird.

16. Zelle, die durch ein Verfahren nach einem der Ansprüche 14 - 15 transformiert wurde.

17. Zusammensetzung mit einem Molekül zum Kompaktieren von DNA und einer Verbindung nach einem der Ansprüche 1-10 oder einem positiv geladenen Vesikel nach Anspruch 11 oder einem Vektor nach Anspruch 12.

18. Zusammensetzung nach Anspruch 17, wobei das Molekül ausgewählt wird aus der Gruppe, bestehend aus Protaminen, Polylysinen und von Histon abgeleiteten Peptiden.

19. Transfektionskit mit einer Zusammensetzung nach Anspruch 17 oder Anspruch 18.

20. Transgene Pflanze oder nicht menschliches Tier, hergestellt aus einer Zelle nach Anspruch 16.

21. Pharmazeutische Zusammensetzung mit einem Vektor nach Anspruch 12 oder einer Zelle nach Anspruch 16.

22. Pharmazeutische Zusammensetzung nach Anspruch 21, die eine Vakzine ist.

23. Pharmazeutische Zusammensetzung nach Anspruch 21 zur Behandlung zellulärer Störungen oder Infektionen.

24. Kosmetische Zusammensetzung mit einem Vektor nach Anspruch 12 oder einer Zelle nach Anspruch 16.

## Revendications

1. Un composé dénommé 3-dodécylamino-N-isopropyl-N'-dodécyl-propionamidine.

2. Un composé selon la revendication 1, ayant un point de fusion de 27-28°C.

3. Un composé selon la revendication 1 ou la revendication 2, ayant un spectre RMN ¹H avec des pics caractéristiques à 3.73, 3.13, 2.79, 2.58, 2.35, 1.47, 1.26, 1.10 et 0.88.

4. Un composé dénommé 3-tétradécylamino-N-isopropyl-N'-tétradécyl-propionamidine.

5. Un composé selon la revendication 4, ayant un point de fusion de 29-30°C.

6. Un composé selon la revendication 4 ou la revendication 5, ayant un spectre RMN ¹H avec des pics caractéristiques à 3.73, 3.14, 2.79, 2.58, 2.34, 1.47, 1.26, 1.10 and 0.88.

7. Un composé dénommé 3-hexadécylamino-N-isopropyl-N'-hexadécyl-propionamidine.

8. Un composé selon la revendication 7, ayant un point de fusion de 38.5-39.5°C.

9. Un composé selon la revendication 7 ou la revendication 8, ayant un spectre RMN ¹H avec des pics caractéristiques à 3.74, 3.14, 2.78, 2.58, 2.34, 1.46, 1.26, 1.10 and 0.88.

10. Un composé dénommé 3-octadécylamino-N-isopropyl-N'-octadécyl-propionamidine.

11. Une vésicule chargée positivement ayant une membrane comprenant un composé selon l'une quelconque des revendications 1 à 10.

12. Un vecteur constitué d'une vésicule chargée positivement selon la revendication 11, ladite vésicule étant associée à au moins une molécule choisie parmi le groupe constitué d'acides nucléiques, de plasmides, de messagers ARN, d'ARN anti-sens, de cADN, d'oligonucléotides synthétiques, de polypeptides, de protéines, de protéines glycosylées, d' agents thérapeutiques et d'agents cosmétiques.

13. Utilisation d'un vecteur selon la revendication 12 dans un sérum.

14. Un procédé pour introduire au moins une molécule choisie parmi le groupe constitué d'acides nucléiques, de plasmides, de messagers ARN, d'ARN anti-sens, decADN, d'oligonucléotides synthétiques, d'antigènes, de polypeptides, de protéines, de protéines glycosylées, d'agents thérapeutiques et d'agents cosmétiques dans une cellule, dans lequel ladite cellule est mise en contact avec ladite molécule et avec un composé selon l'une quelconque des revendications 1 à 10 ou une vésicule chargée positivement selon la revendication 11 ou un vecteur selon la revendication 12, dans lequel la cellule est traitée *in vivo* pour produire des microorganismes recombinants ou des plantes.

15. Le procédé de la revendication 14, dans lequel la cellule est traitée *in vitro*.

16. Une cellule transformée par un procédé selon l'une quelconque des revendications 14 à 15.

17. Une composition comprenant une molécule pour compacter de l'ADN et un composé selon l'une quelconque des revendications 1 à 10 ou une vésicule chargée positivement selon la revendication 11 ou un vecteur selon la revendication 12.

18. Une composition selon la revendication 17, dans laquelle ladite molécule est choisie parmi le groupe constitué de protamines, de polylysines et de peptides dérivés de l'histone.

19. Un kit de transfection comprenant une composition selon la revendication 17 ou à la revendication 18.

20. Une plante transgénique ou un animal non-humain produit à partir d'une cellule selon la revendication 16.

21. Une composition pharmaceutique comprenant un vecteur selon la revendication 12 ou une cellule selon la revendication 16.

22. Une composition pharmaceutique selon la revendication 21, sous la forme d'un vaccin.

23. Une composition pharmaceutique selon la revendication 21 pour le traitement de désordres ou infections cellulaires.

24. Une composition cosmétique comprenant un vecteur selon la revendication 12 ou une cellule selon la revendication 16.
